# EUROPEAN PATENT APPLICATION

(11) **EP 4 302 770 A1**
(43) Date of publication of application: **10.01.2024**
(21) Application number: 22183717.2
(22) Date of filing: 08.07.2022
(51) Int. Cl.: A61K 35/28, A61K 38/48, A61K 38/47, A61P 7/00, A61P 7/06, A61P 35/00, A61P 35/02

(54) **REGIMEN FOR ENZYMATIC DESENSITISATION**

(71) Applicant: Hansa Biopharma AB, 220 07 Lund (SE)
(72) Inventor: The designation of the inventor has not yet been filed
(74) Representative: J A Kemp LLP

(57) **Abstract**

The present invention relates to a conditioning regimen for a cell transplant to a sensitized subject, which is optionally of hematopoietic stem / progenitor cells (HSPC). The invention also relates to methods comprising administration of a cell transplant to a sensitized subject in accordance with the conditioning regimen, for the prevention or treatment of immune rejection of a cell transplant, for the prevention or treatment of engraftment failure or poor graft function of a cell transplant, or for the prevention or treatment of a disease or condition that is treated or prevented by a cell transplant. The invention also provides methods for determining whether a patient is sensitized and hence that the conditioning regimen should be used.

## Description

### Field of the Invention

The present invention relates to a conditioning regimen for a cell transplant to a sensitized subject, which is optionally of hematopoietic stem / progenitor cells (HSPC). The invention also relates to methods comprising administration of a cell transplant to a sensitized subject in accordance with the conditioning regimen, for the prevention or treatment of immune rejection of a cell transplant, for the prevention or treatment of engraftment failure or poor graft function of a cell transplant, or for the prevention or treatment of a disease or condition that is treated or prevented by a cell transplant. The invention also provides methods for determining whether a patient is sensitized and hence that the conditioning regimen should be used.

### Background of the Invention

The complex immunology involved in the transplant of cells, such as HSPC, can be problematic, particularly if there is sensitization to donor antigens prior to transplantation. The presence of donor and recipient immune systems can lead to acute and chronic rejection with both humeral and cellular components. Donor-specific antibodies can be particularly problematic, particularly if present at high levels. Vigorous host versus graft reactions (HVG) and graft versus host disease (GVHD) are both observed. Often, the transplanted cells fail to successfully engraft in the recipient. Current methods seek to address these problems by pre- and post-transplant immunosuppression. Steps carried out pre-transplant may be referred to as a conditioning regimen and may include treatments that are not solely immunosuppressive. For example, radiation may be used to deplete some or all of the existing bone marrow cells in the recipient, creating space for engraftment of the transplanted cells. However, lymphocyte depleting agents are often used. Using high doses of these agents leads to a desired reduction of GvHD, but at the cost of delayed immune reconstitution or even increased host engraftment failure (because the agents also act on the transplanted cells), thereby enhancing the risk of severe infectious complications and reducing overall survival (OS). There is a need for improved conditioning regimens for the transplant of cells, particularly for subjects who have been sensitized, for example if they have developed donor-specific antibodies.

### Summary of the Invention

Conditioning regimens have been described in which enzymes are used (typically pre-transplant) to inactivate donor specific antibodies (DSA) in a cell transplant recipient (see e.g. WO2021/233924). Similar enzymes have been used (again typically before transplant) to inactivate DSA in sensitized solid organ transplant recipients (see, e.g. Jordan et al, Transplantation; Aug 2021, 105(8): 1808-1817). In both cases, the intended purpose is the same: to improve the likelihood of a successful transplant.

The present inventors have determined that sensitized subjects face additional difficulties with a cell transplant relative to a solid organ transplant, and that this may require specific adaptations of the protocol by which such enzymes are administered. This is because cell transplants typically comprise non-adherent cells. Such cells, and particularly HSPC, are likely to be more sensitive to antibody-dependent cell-mediated cytotoxicity (ADCC), antibody-dependent cellular phagocytosis (ADCP), and complement-dependent cytotoxicity (CDC) compared to adherent cells and especially compared to complete organs such as kidney. HSPC are typically dosed at approximately 5 × 10⁶ CD34+ cells/kg. In an adult of average weight (70 kg) this equals approximately 3.5 × 10⁸ CD34+ cells being dosed. Only a fraction of these cells are true pluripotent cells with the capacity to generate a functioning bone marrow. By contrast, when transplanting an entire organ like a kidney, all the needed cells for the graft to function are already available within the transplant itself, and the number of cells is 100 fold higher than a transplant of HSPC. A kidney constitutes roughly 2000-4000 glomeruli with a total of approximately 100 × 10⁸ glomerulus cells (and additionally supportive cell types) thus there is a major difference in the number of target cells which may be bound by DSA, and also in the relative importance of each of these cells to successful transplant. In other words, DSA-levels shown to be safe in the solid organ transplant setting may have a very negative impact on the survival of a cell transplant. As such a conditioning regimen for cell transplant must apply different criteria to a conditioning regimen for organ transplant.

There are additional challenges relating to timing, in that a cell transplant is likely to remain vulnerable to DSA for a longer period than a solid organ, because the transplanted cells will cross-circulate between bone marrow and peripheral blood repeatedly and are the only donor cells present until engraftment has occurred. In this period, they are particularly vulnerable to DSA because they are comparatively scarce - likely the only cells present with donor antigens, i.e. expressing foreign HLA. The present inventors show that this period of vulnerability lasts until at least approximately 14 days post-transplant. At this stage, a successful HSPC engraftment may begin to produce neutrophils and platelets, which start to appear in the blood from around 2-3 weeks from transplant. The neutrophils and platelets also have donor HLA, and thus may bind to DSA reducing the quantity available to bind to the transplanted HSPC. Data in certain sensitized solid organ transplant recipients suggests that DSA levels can begin to rebound within this timeframe following treatment with an inactivating enzyme. This suggests that the conditioning regimen with antibody inactivating enzymes that is suitable for solid organ transplants may not be suitable for cell transplants. In other words, cell transplant to sensitized subjects requires a particular form of conditioning regimen including such enzymes, because the cell transplant recipient may require a further dose of the enzyme in order to ensure that DSA remains below a safe threshold. Such a conditioning regimen is provided by the present invention.

The present invention provides a conditioning regimen for the transplant of a cell to a subject, comprising:
a. administering to the subject a dose of an enzyme which inactivates serum IgG molecules in the subject;
b. subsequently administering the transplant to the subject;
c. subsequently administering to the subject a further dose of an enzyme which inactivates serum IgG molecules in the subject, which is optionally the same enzyme.

The present invention also provides a method for the prevention or treatment of immune rejection of a cell transplant to a sensitized subject, the method comprising administering a cell transplant to the subject in accordance with the conditioning regimen of the invention.

The present invention also provides a method for the prevention or treatment of engraftment failure or poor graft function of a cell transplant to a sensitized subject, the method comprising administering a cell transplant to the subject in accordance with the conditioning regime of the invention.

The present invention also provides a method for the prevention or treatment of a disease or condition in a sensitized subject, which disease or condition is prevented or treated by cell transplant, the method comprising administering a cell transplant to the subject in accordance with the conditioning regime of the invention. The disease or condition may be selected from Hematological malignancies; Solid tumor cancers; Hematologic diseases; Anemias; Myeloproliferative disorders; Metabolic disorders; Environmentally-induced diseases; Viral diseases; Autoimmune diseases; Lysosomal storage disorders; and Immunodeficiencies.

### Brief Description of the Figures

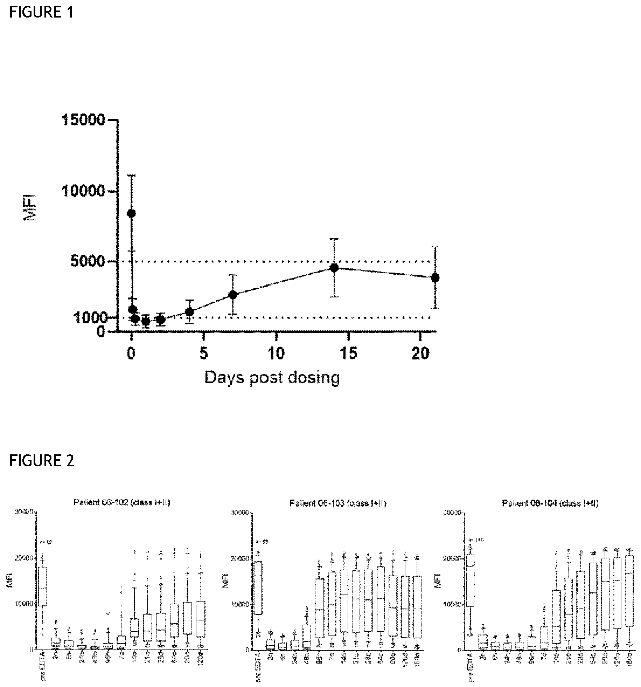

Fig. 1. shows the mean of all DSA levels above 1000 MFI before administration of enzyme (pre-dose) in a human study. Mean + 95% CI plotted. Dotted lines mark 1000 MFI (often used to define a positive DSA) and 5000 MFI (above this level there is an increased risk of complement-fixation).
**Fig. 2** shows pre-dose MFI levels (combined anti HLA class I and II levels) in three individual patients in the study, illustrating the variation in response and duration of response for different anti-HLA antibodies.

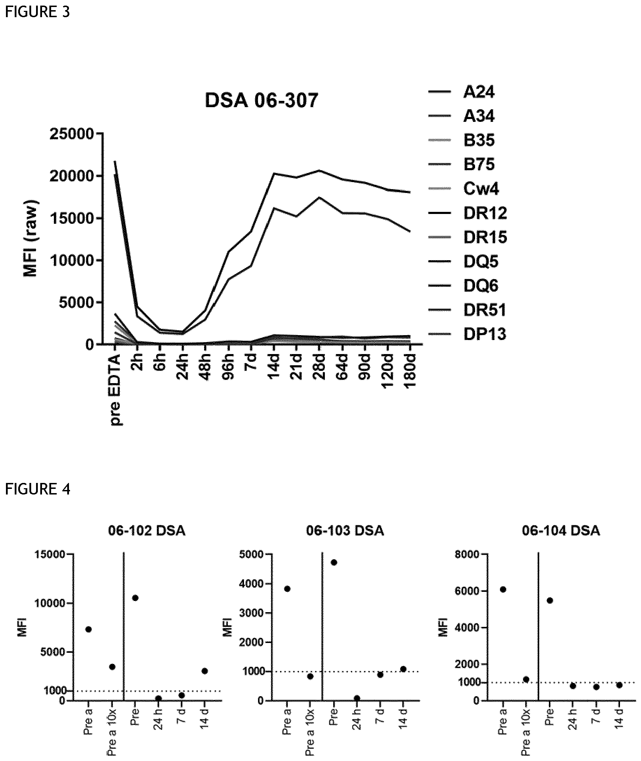

**Fig. 3****.** shows DSA-rebound pattern for an individual patient with two very high DSAs at predose and a slight increase in MFI already within 48 hours post-dose, from which level it continued to increase rapidly during the next days.
**Fig. 4****.** shows DSA levels in pre-dose samples (Pre a) and 10-fold diluted pre-dose (Pre a 10×) samples (left part of each graph) compared to pre-dose (pre) samples and different post-dose time-points, 24hours, 7 days, and 14 days, in three individual patients in the same study as above. Each patient had one DSA above 1000 at pre-dose. Dotted lines mark 1000 MFI.
The Pre a 10× samples are clearly seen to have MFI in the same range as the 14d post dose sample, supporting the predictive power of the Pre a 10× sample.

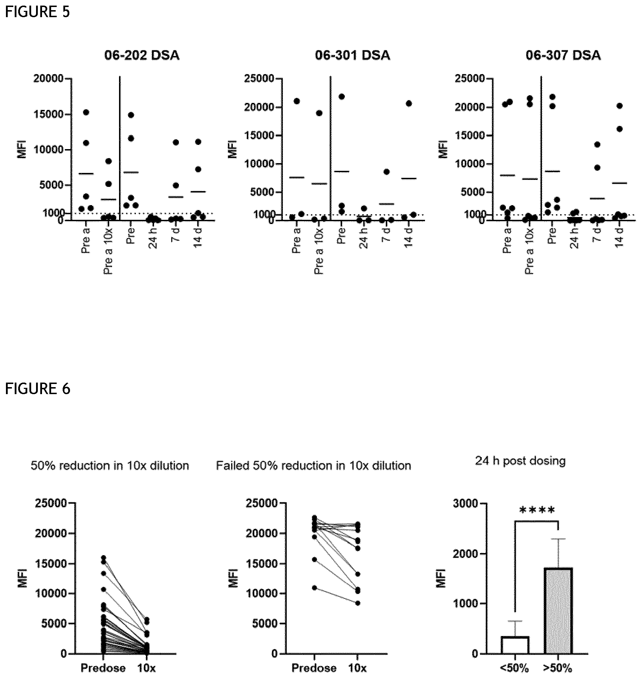

**Fig. 5****.** shows DSA levels in pre-dose samples (Pre a) and 10-fold diluted pre-dose (Pre a 10×) samples (left part of each graph) compared to pre-dose (pre) samples and different post-dose time-points, 24hours, 7 days, and 14 days, in three further individual patients in the same study as above. Each patient had several DSA above 1000 at pre-dose. Dotted lines mark 1000 MFI. The Pre a 10× samples are clearly seen to have MFI in the same range as the 14d post dose sample, again supporting the predictive power of the Pre a 10× sample.
**Fig. 6****.** shows DSA levels in pre-dose samples and 10-fold diluted pre-dose (10×) samples sorted according to if they were reduced at least 50% in the 10-fold diluted sample (left figure, n=45) or were not reduced at least 50% (middle figure, n=14). The right figure shows the actual MFI results 24 hours after dosing of the IgG-degrading enzyme in both patient groups (mean + 95% CI plotted, Mann-Whitney, p<0.0001).

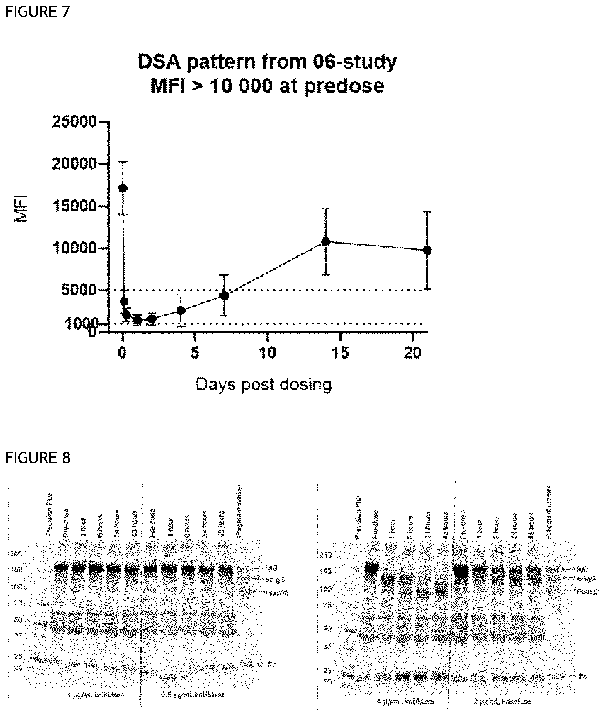

**Fig. 7****.** shows the mean of all DSA levels above 10,000 MFI before administration of enzyme (pre-dose) in the same human study. Mean + 95% CI plotted. Dotted lines mark 1000 MFI (often used to define a positive DSA) and 5000 MFI (above this level there is an increased risk of complement-fixation).
**Fig 8**. shows SDS-PAGE separation of a serum pool (n = 100) treated with 0.5, 1, 2 or 4 µg/mL imlifidase for 1 to 48 hours. It is demonstrated that a concentration of around 2 µg/mL imlifidase was needed to start the cleavage in serum and concentrations below this did not result in any cleavage despite prolonged incubation.

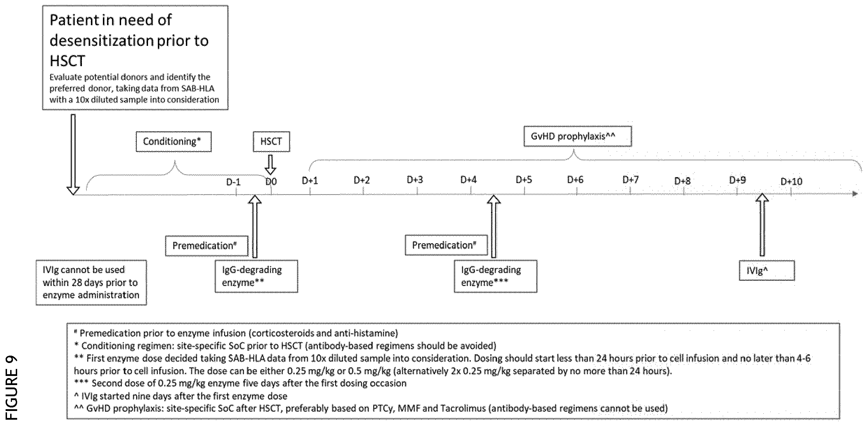

**Fig. 9** shows the timeline of an exemplary conditioning regimen.

### Brief Description of the Sequences

SEQ ID NO: 1 is the full sequence of IdeS including N terminal methionine and signal sequence. It is also available as NCBI Reference sequence no. WP_010922160.1
SEQ ID NO: 2 is the mature sequence of IdeS, lacking the N terminal methionine and signal sequence. It is also available as Genbank accession no. ADF13949.1
SEQ ID NO: 3 is the full sequence of IdeZ including N terminal methionine and signal sequence. It is also available as NCBI Reference sequence no. WP_014622780.1.
SEQ ID NO: 4 is the mature sequence of IdeZ, lacking the N terminal methionine and signal sequence.
SEQ ID NO: 5 is the sequence of a hybrid IdeS/Z. The N terminus is based on IdeZ lacking the N terminal methionine and signal sequence.
SEQ ID NOs: 6 to 25 are the sequences of exemplary proteases for use in the methods of the invention.
SEQ ID NO: 26 is the sequence of an IdeS polypeptide. Comprises the sequence of SEQ ID NO: 2 with an additional N terminal methionine and a histidine tag (internal reference pCART124).
SEQ ID NO: 27 is the sequence of an IdeZ polypeptide. Comprises the sequence of SEQ ID NO: 4 with an additional N terminal methionine and a histidine tag (internal reference pCART144).
SEQ ID NO: 28 is the sequence of an IdeS/Z polypeptide. Comprises the sequence of SEQ ID NO: 5 with an additional N terminal methionine and a histidine tag (internal reference pCART145).
SEQ ID NO: 29 is the contiguous sequence PLTPEQFRYNN, which corresponds to positions 63-73 of SEQ ID NO: 3.
SEQ ID NO: 30 is the contiguous sequence PPANFTQG, which corresponds to positions 58-65 of SEQ ID NO: 1.
SEQ ID NO: 31 is the contiguous sequence DDYQRNATEAYAKEVPHQIT, which corresponds to positions 35-54 of SEQ ID NO: 3.
SEQ ID NO: 32 is the contiguous sequence DSFSANQEIRYSEVTPYHVT, which corresponds to positions 30-49 of SEQ ID NO: 1.
SEQ ID NOs: 33 to 55 are nucleotide sequences encoding proteases set out above.
SEQ ID NOs: 56 to 69 are the sequences of exemplary proteases for use in the methods of the invention.
SEQ ID NO: 70 is the contiguous sequence NQTN, which corresponds to positions 336-339 of SEQ ID NO: 1.
SEQ ID NO: 71 is the contiguous sequence DSFSANQEIR YSEVTPYHVT, which corresponds to positions 30-49 of SEQ ID NO: 1.
SEQ ID NOs: 72 to 86 are nucleotide sequences encoding polypeptides disclosed herein. SEQ ID NO: 87 is the sequence SFSANQEIRY SEVTPYHVT, which corresponds to positions 31-49 of SEQ ID NO: 1.
SEQ ID NO: 88 is the sequence DYQRNATEAY AKEVPHQIT, which corresponds to positions 36-54 of the IdeZ polypeptide NCBI Reference Sequence no WP_014622780.1. SEQ ID NO: 89 is the sequence DDYQRNATEA YAKEVPHQIT, which may be present at the N terminus of a polypeptide of the invention.
SEQ ID NO: 90 is the mature sequence of EndoS (Endoglycosidase of S. pyogenes).
SEQ ID NO: 91 and SEQ ID NO: 92 are further exemplary proteases for use in the methods of the invention. SEQ ID NO: 92 is the same as SEQ ID NO: 91, except it lacks the first twenty residues at the N terminus of SEQ ID NO: 1 consisting of the contiguous sequence DDYQRNATEAY AKEVPHQIT.

### Detailed Description of the Invention

### General

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "a polypeptide" includes "polypeptides", and the like.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including both D or L optical isomers, and amino acid analogs and peptidomimetics.

The terms "patient" and "subject" are used interchangeably and typically refer to a human. References to IgG typically refer to human IgG unless otherwise stated.

Amino acid identity as discussed above may be calculated using any suitable algorithm. For example the PILEUP and BLAST algorithms can be used to calculate identity or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al*, supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001. Alternatively, the UWGCG Package provides the BESTFIT program which can be used to calculate identity (for example used on its default settings) (Devereux et al (1984) Nucleic Acids Research 12, 387-395).

All publications, patents and patent applications cited herein, whether supra or infra, are hereby incorporated by reference in their entirety.

### Conditioning regimen

The present invention provides a conditioning regimen for a cell transplant to a sensitized subject, comprising:
a. administering to the subject a dose of an enzyme which inactivates serum IgG molecules in the subject;
b. subsequently administering the transplant to the subject;
c. subsequently administering to the subject a further dose of an enzyme which inactivates serum IgG molecules in the subject, which is optionally the same enzyme.

### Conditioning regimen - enzyme

The amount of said enzyme administered in steps a and c is preferably sufficient to inactivate all or substantially all IgG molecules present in the serum of the subject. If necessary, more than one IgG-inactivating enzyme can be administered in combination, including simultaneously or sequentially, in any order.

The term "serum IgG molecule(s)" or "IgG molecule(s) present in the serum" refers to any gamma immunoglobulin (IgG1, IgG2, IgG3 and IgG4) molecule which is present in human tissue or in circulation prior to a method of the invention being carried out. Such IgG molecules may have been produced endogenously from an individual's B-cells or may be exogenous gamma immunoglobulins which have been administered to a subject prior to the method of the invention being carried out - including any therapeutic IgG molecule of any origin. Inactivation of serum IgG typically means a reduction in the Fc receptor interaction of IgG molecules. The term "Fc receptor" refers to Fc gamma immunoglobulin receptors (FcyRs) which are present on cells. In humans, FcyR refers to one, some, or all of the family of receptors comprising FcyRI (CD64), FcyRIIA (CD32A), FcγRIIB (CD32B), FcyRIIC (CD32C), FcyRIIIA (CD16a) and FcyRIIIB (CD16b). As used herein, the term FcyR includes naturally occurring polymorphisms of FcyRI (CD64), FcyRIIA (CD32A), FcγRIIB (CD32B), FcyRIIC (CD32C), FcyRIIIA (CD16a) and FcγRIIIB (CD16b). Inactivation of IgG can also reduce complement activation, which is also fundamentally dependent on interactions through an intact IgG molecule.

The enzyme used in the method of the invention may be any enzyme which inactivates serum IgG, but is typically an IgG cysteine protease which cleaves IgG such that the antigen binding domains and Fc interacting domains are separated from each other. In such cases, Fc receptor interaction of serum IgG molecules is reduced because the quantity of intact IgG molecules in the serum is reduced. As another example, the enzyme may be an IgG endoglycosidase which cleaves a glycan structure on the Fc interacting domain of IgG, particularly the N-linked bi-antennary glycan at position Asn-297 (Kabat numbering). This glycan structure has a critical role in Fc receptor binding and complement activation. Thus, when it is wholly or partially removed by a protein, this will lead to reduced Fc receptor binding or complement activation by an otherwise intact IgG molecule, as well as also reduced recycling/half-life due to reduced binding to the FcRn. Enzymes suitable for use in the conditioning regimen are discussed in more detail in subsequent sections below.

The enzyme is preferably administered by intravenous infusion, but may be administered by any suitable route including, for example, intradermal, subcutaneous, percutaneous, intramuscular, intra-arterial, intraperitoneal, intraarticular, intraosseous or other appropriate administration routes. The amount of the enzyme that is administered may be between 0.01 mg/kg BW and 2 mg/kg BW, between 0.01 mg/kg BW and 1 mg/kg BW, between 0.01 mg/kg BW and 0.5 mg/kg BW, between 0.01 and 0.3 mg/kg BW, preferably between 0.1 and 0.3 mg/kg BW, most preferably between 0.2 and 0.3 mg/kg BW. A particularly preferred dose is approximately 0.25 mg/kg BW. Under certain circumstances (as outlined below) a higher preferred dose may be adopted, particularly for an initial dose of enzyme. The higher dose is typically up to approximately double the preferred dose of 0.25 mg/kg BW, i.e. it is up to 0.5 mg/kg BW. Thus, preferred doses may be from about 0.25 mg/kg BW to about 0.5 mg/kg BW, or from about 0.3 mg/kg BW to about 0.5 mg/kg BW. Higher doses (particularly higher initial doses) may be administered in a single administration, or as two or more separate administrations that cumulatively add up to the higher dose. In such cases, the separate administrations are typically administered within a time interval of upto 24 hours, preferably upto 12 hours. The dose is considered to be complete with the final said administration in the period.

The enzyme may be administered on multiple occasions to the same subject, provided that the quantity of anti-drug antibody (ADA) in the serum of the subject which is capable of binding to the enzyme does not exceed a threshold determined by the clinician. The amount of enzyme administered may be increased should a clinician consider this to be appropriate. The quantity of ADA in the serum of the subject which is capable of binding to the protease may be determined by any suitable method, such as an agent specific CAP FEIA (ImmunoCAP) test, a titre assay or a functional assay. If ADA in the subject exceed said threshold, the condition regimen may include administration of an alternative enzyme.

The enzyme may be any of the following:

### IgG cysteine proteases

The IgG cysteine protease for use with the invention is specific for IgG. The IgG cysteine protease may be from a Streptococcus bacterium, such as Streptococcus pyogenes Streptococcus equi or Streptococcus zooepidemicus, optionally wherein said enzyme is a IdeS, MAC2, SpeB, IdeZ or IgdE polypeptide. In preferred embodiments, the protease for use in the methods of the invention is IdeS (Immunoglobulin G-degrading enzyme of S. *pyogenes*), otherwise known as imlifidase, or a homologue or variant thereof. IdeS is an extracellular cysteine protease produced by the human pathogen *S. pyogenes.*

IdeS was originally isolated from a group A *Streptococcus* strain of serotype M1, but the *ides* gene has now been identified in all tested group A *Streptococcus* strains. IdeS has an extraordinarily high degree of substrate specificity, with its only identified substrate being IgG. IdeS catalyses a single proteolytic cleavage in the lower hinge region of the heavy chains of all subclasses of human IgG. IdeS also catalyses an equivalent cleavage of the heavy chains of some subclasses of IgG in various animals. IdeS efficiently cleaves IgG to Fc and F(ab')₂ fragments via a two-stage mechanism. In the first stage, one (first) heavy chain of IgG is cleaved to generate a single cleaved IgG (scIgG) molecule with a non-covalently bound Fc/2 molecule. The scIgG molecule is effectively an intermediate product which retains the remaining (second) heavy chain of the original IgG molecule. In the second stage of the mechanism this second heavy chain is cleaved by IdeS to release a F(ab')₂ fragment and a homodimeric Fc fragment. These are the products generally observed under physiological conditions. Under reducing conditions the F(ab')₂ fragment may dissociate to two Fab' fragments and the homodimeric Fc may dissociate into its component monomers. IdeS has been shown to be particularly effective at cleaving IgG in humans. The entire plasma IgG-pool is cleaved within minutes of dosing with IdeS, and IgG levels in blood remain low for more than a week until newly synthesized IgG appeared in plasma. This demonstrates that the entire extracellular IgG pool and not only the plasma pool (i.e. serum IgG molecules) is cleaved by IdeS (Winstedt et al; PloS One 2015; 10(7): e0132011).

SEQ ID NO: 1 is the full sequence of IdeS including the N terminal methionine and signal sequence. It is also available as NCBI Reference sequence no. WP_010922160.1. SEQ ID NO: 2 is the mature sequence of IdeS, lacking the N terminal methionine and signal sequence. It is also available as Genbank accession no. ADF13949.1. A variant of IdeS is the protein MAC2, the full sequence of which is available as Genbank Accession no. AFC67907.1. This protein (with or without signal sequence) is also suitable for use in the methods described herein.

In alternative embodiments, the protease for use in the methods of the invention is IdeZ, which is a IgG cysteine protease produced by *Streptococcus equi ssp. Zooepidemicus,* a bacterium predominantly found in horses. SEQ ID NO: 3 is the full sequence of IdeZ including N terminal methionine and signal sequence. It is also available as NCBI Reference sequence no. WP_014622780.1. SEQ ID NO: 4 is the mature sequence of IdeZ, lacking the N terminal methionine and signal sequence.

In alternative embodiments, the protease for use in the methods of the invention is a hybrid IdeS/Z, such as that of SEQ ID NO: 5. The N terminus is based on IdeZ lacking the N terminal methionine and signal sequence.

In preferred embodiments, the protease for use in the invention may comprise or consist of SEQ ID NO: 2, 4 or 5. Proteases for use in the invention may comprise an additional methionine (M) residue at the N terminus and/or a tag at the C terminus to assist with expression in and isolation from standard bacterial expression systems. Suitable tags include a histidine tag which may be joined directly to the C terminus of a polypeptide or joined indirectly by any suitable linker sequence, such as 3, 4 or 5 glycine residues. The histidine tag typically consists of six histidine residues, although it can be longer than this, typically up to 7, 8, 9, 10 or 20 amino acids or shorter, for example 5, 4, 3, 2 or 1 amino acids.

In further preferred embodiments, the protease for use in the invention may comprise, consist essentially, or consist of the sequence of any one of SEQ ID NOs: 6 to 25. These sequences represent IdeS and IdeZ polypeptides with increased protease activity and/or reduced immunogenicity. Each of SEQ ID NOs: 6 to 25 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 56 to 69, 91 or 92. These sequences represent IdeS polypeptides with increased protease activity and/or reduced immunogenicity. Each of SEQ ID NOs: 56 to 69, 91 or 92 may optionally include an additional methionine at the N terminus and/or a histidine tag at the C terminus. The histidine tag preferably consists of six histidine residues. The histidine tag is preferably linked to the C terminus by a linker of 3× glycine or 5x glycine residues.

In further preferred embodiments, the protease for use in the invention may comprise, consist essentially, or consist of the sequence of any one of SEQ ID NOs: 6 to 25, optionally with up to 3 (such as 1, 2 or 3) amino acid substitutions. Each of SEQ ID NOs: 6 to 25 and variants thereof may optionally include an additional methionine at the N terminus and/or a histidine tag at the C terminus.

In further preferred embodiments, the protease for use in the invention may comprise, consist essentially, or consist of the sequence of any one of SEQ ID NOs: 56 to 69, 91 or 92, optionally with up to 3 (such as 1, 2 or 3) amino acid substitutions. Each of SEQ ID NOs: 56 to 69, 91 or 92 and variants thereof may optionally include an additional methionine at the N terminus and/or a histidine tag at the C terminus.

The polypeptide of the invention is typically at least 100, 150, 200, 250, 260, 270, 280, 290, 300 or 310 amino acids in length. The polypeptide of the invention is typically no larger than 400, 350, 340, 330, 320 or 315 amino acids in length. It will be appreciated that any of the above listed lower limits may be combined with any of the above listed upper limits to provide a range for the length the polypeptide of the invention. For example, the polypeptide may be 100 to 400 amino acids in length, or 250 to 350 amino acids in length. The polypeptide is preferably 290 to 320 amino acids in length, most preferably 300 to 315 amino acids in length.

The primary structure (amino acid sequence) of a protease of the invention is based on the primary structure of IdeS, IdeZ or IdeS/Z, specifically the amino acid sequence of SEQ ID NO: 2, 4 or 5, respectively. The sequence of a protease of the invention may comprise a variant of the amino acid sequence of SEQ ID NO: 2, 4 or 5, which is at least 80% identical to the amino acid sequence of SEQ ID NO: 2, 4 or 5. The variant sequence may be at least 80%, at least, 85%, preferably at least 90%, at least 95%, at least 98% or at least 99% identical to the sequence of SEQ ID NO: 2, 4 or 5. The variant may be identical to the sequence of SEQ ID NO: 2, 4 or 5 apart from the inclusion of one or more of the specific modifications identified in WO2016/128558, WO2016/128559 or WO2021/233911. Identity relative to the sequence of SEQ ID NO: 2, 4 or 5 can be measured over a region of at least 50, at least 100, at least 200, at least 300 or more contiguous amino acids of the sequence shown in SEQ ID NO: 2, 4 or 5, or more preferably over the full length of SEQ ID NO: 4 or 5.

The protease for use in the invention may be an IdeS, IdeZ or IdeS/Z polypeptide that comprises a variant of the amino acid sequence of SEQ ID NO:, 2, 4 or 5 in which modifications, such as amino acid additions, deletions or substitutions are made relative to the sequence of SEQ ID NO: 2, 4 or 5. Such modifications are preferably conservative amino acid substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art.

IgG cysteine protease activity may be assessed by any suitable method, for example by incubating a polypeptide with a sample containing IgG and determining the presence of IgG cleavage products. Suitable methods are described in the WO2016/128559. Suitable assays include an ELISA-based assay, such as that which is described in WO2016/128559. In such an assay, the wells of an assay plate will typically be coated with an antibody target, such as bovine serum albumin (BSA). Samples of the polypeptide to be tested are then added to the wells, followed by samples of target-specific antibody that is antibody specific for BSA in this example. The polypeptide and antibody are allowed to interact under conditions suitable for IgG cysteine protease activity. After a suitable interval, the assay plate will be washed and a detector antibody which specifically binds to the target-specific antibody will be added under conditions suitable for binding to the target-specific antibody. The detector antibody will bind to any intact target-specific antibody that has bound to the target in each well. After washing, the amount of detector antibody present in a well will be proportional to the amount of target-specific antibody bound to that well. The detector antibody may be conjugated directly or indirectly to a label or another reporter system (such as an enzyme), such that the amount of detector antibody remaining in each well can be determined. The higher the potency of the tested polypeptide that was in a well, the less intact target-specific antibody will remain and thus there will be less detector antibody. Typically, at least one well on a given assay plate will include IdeS instead of a polypeptide to be tested, so that the potency of the tested polypeptides may be directly compared to the potency of IdeS. IdeZ and IdeS/Z may also be included for comparison.

Other assays may determine the potency of a tested polypeptide by directly visualizing and/or quantifying the fragments of IgG which result from cleavage of IgG by a tested polypeptide. An assay of this type is also described in WO2016/128559. Such an assay will typically incubate a sample of IgG with a test polypeptide (or with one or more of IdeS, IdeZ and IdeS/Z as a control) at differing concentrations in a titration series. The products which result from incubation at each concentration are then separated using gel electrophoresis, for example by SDS-PAGE. Whole IgG and the fragments which result from cleavage of IgG can then be identified by size and quantified by the intensity of staining with a suitable dye. The greater the quantity of cleavage fragments, the greater the potency of a tested polypeptide at a given concentration. A polypeptide of the invention will typically produce detectable quantities of cleavage fragments at a lower concentration (a lower point in the titration series) than IdeZ and/or IdeS. This type of assay may also enable the identification of test polypeptides that are more effective at cleaving the first or the second heavy chain of an IgG molecule, as the quantities of the different fragments resulting from each cleavage event may also be determined. A polypeptide of the invention may be more effective at cleaving the first chain of an IgG molecule than the second, particularly when the IgG is an IgG2 isotype. A polypeptide of the invention may be more effective at cleaving IgG1 than IgG2.

### IgG endoglycosidases

The enzyme may have IgG endoglycosidase activity, preferably cleaving the glycan moiety at Asn-297 (Kabat numbering) in the Fc region of IgG. An example of such a protein is EndoS (Endoglycosidase of *S. pyogenes*). EndoS hydrolyzes the β-1,4-di-*N-*acetylchitobiose core of the asparagine-linked glycan of normally-glycosylated IgG. The mature sequence of EndoS is provided as SEQ ID NO: 90. The agent may be a protein comprising or consisting of the amino acid sequence of SEQ ID NO: 90, or may be a homologue thereof from an alternative bacterium, such as Streptococcus equi or Streptococcus zooepidemicus, or Corynebacterium pseudotuberculosis, Enterococcus faecalis, or Elizabethkingia meningoseptica. The agent may be CP40, EndoE, or EndoF₂.

Alternatively the agent may be a variant of the EndoS protein which comprises or consists of any amino acid sequence which has at least 80%, 85%, 90% or 95% identity with SEQ ID NO: 90 and has IgG endoglycosidase activity. A variant of the EndoS protein may comprise or consist of an amino acid sequence in which up to 1, 2, 3, 4, 5, 10, 20, 30, 40, 50, 60, 70, 80, 90, or more, amino acid substitutions, insertions or deletions have been made relative to the amino acid sequence of SEQ ID NO: 90, provided the variant has IgG endoglycosidase activity. Said amino acid substitutions are preferably conservative. Conservative substitutions are as defined in the preceding section.

Alternatively the agent may be a protein which comprises or consists of a fragment of SEQ ID NO: 90 and has IgG endoglycosidase activity, preferably wherein said fragment is 400 to 950, 500 to 950, 600 to 950, 700 to 950 or 800 to 950 amino acids in length. A preferred fragment consists of amino acids 1 to 409 of SEQ ID NO: 90, which corresponds to the enzymatically active α-domain of EndoS generated by cleavage by the streptococcal cysteine proteinase SpeB. The fragment may be created by the deletion of one or more amino acid residues of the amino acid sequence of SEQ ID NO: 90. Up to 1, 2, 3, 4, 5, 10, 20, 30, 40, 50, 100, 200, 300, 400, 500 or 550 residues may be deleted, or more. The deleted residues may be contiguous with other.

Any fragment or variant of SEQ ID NO: 90 preferably includes residues 191 to 199 of SEQ ID NO: 90, i.e. Leu-191, Asp-192, Gly-193, Leu-194, Asp-195, Val-196, Asp-197, Val-198 and Glu-199 of SEQ ID NO: 90. These amino acids constitute a perfect chitinase family 18 active site, ending with glutamic acid. The glutamic acid in the active site of chitinases is essential for enzymatic activity. Most preferably, therefore, a variant of SEQ ID NO: 90 contains Glu-199 of SEQ ID NO: 90. The variant of SEQ ID NO: 90 may contain residues 191 to 199 of SEQ ID NO: 90 having one or more conservative substitutions, provided that the variant contains Glu-199 of SEQ ID NO: 90.

The conditioning regimen of the invention requires a second dose of IgG cleaving enzyme. Given that many of the enzymes with this function are of bacterial (often pathogenic bacterial) origin, the second dose could be problematic due to patient immune responses to it. For example, imlifidase is a bacterial enzyme derived from a *S pyogenes*, and the majority of humans have already been exposed to it and have developed an immunological memory against. This can result in fast and sometimes very strong anti-drug antibody response. This makes repeat dosing less feasible, though after dosing most humans exposed to imlifidase in clinical trials did not regain detectable anti-drug antibodies in circulation prior to day 7 post dosing, and so a second dose of imlifidase in accordance with the present invention should be tolerated. Nonetheless, the response beyond this timepoint has been very individual and most developed a robust response by day 14 (data not shown). As such, it may be preferred to use alternative enzymes which are less immunogenic in human subjects. Examples include the IgG cysteine protease polypeptides of SEQ ID NOs: 6 to 25, 56 to 69, 91 or 92 (or variants thereof as described above), the properties of which are disclosed in detail in WO2016/128558, WO2016/128559 and WO2021/233911.

### Conditioning regime -timing of enzyme administration

The present invention requires the administration of IgG-inactivating enzyme both before and after transplant - in step a and step c. The timing of the two steps is intended to increase the time period in which DSA levels are kept low, such that transplant rejection, engraftment failure, or poor graft function are less likely to occur. The invention arises from the discovery that in certain patients, the recovery of DSA after a first dose of IgG-inactivating enzyme is surprisingly fast, and could reach levels which are problematic given the particular vulnerability of a cell transplant as compared to a solid organ transplant. As is demonstrated in the Examples, the inventors have determined that in such patients, referred to as sensitized patients, DSA recovery may surprisingly reach problematic levels within 3-14 days, and potentially around 3 to 7 days after a first dose of IgG-inactivating enzyme. Accordingly, the conditioning regimen of the present invention includes a second dose of enzyme in step c. Step c may take place between about 3 and about 14 days after step a, that is at about 3, 4, 5, 6, 7, 8, 9, 10, 11, 12, 13, or 14 days after step a. Preferably, step c takes place between about 3 and about 7 days after step a, more preferably between about 4 and about 6 days after step a. Step c may take place at about 5 days after step a. Reference to a day means a period of 24 hours. Thus about 1 day is equivalent to about 24 hours and the terms may be used interchangeably. Thus, the above times can alternatively be expressed as follows. Step c may take place between about 72 hours and about 336 hours after step a, that is at about 72, 96, 120, 144, 168, 192, 216, 240, 264, 288, 312, or 336 hours after step a. Preferably, step c takes place between about 72 hours and about 168 hours after step a, more preferably between about 96 hours and about 144 hours after step a. Step c may take place at about 120 hours after step a.

### Conditioning regime - selection of patients

The present invention is intended to improve transplant success in patients that are sensitized to donor specific antigens. Sensitivity is typically determined by the presence of donor specific antibody (DSA) in the patient. This may be measured in a sample obtained from the patient, typically a fluid sample, such as a serum or a plasma sample. The presence of DSA may be determined by a positive read-out in a solid-phase assay, typically a solid-phase bead assay (e.g. single antigen bead (SAB)-HLA assay, with or without assessing complement fixation), or a cell-based flow cytometry assay (flow cytometry crossmatch), or a cell-based lymphocytotoxic test (e.g. complement dependent cytotoxicity (CDC) assay, with or without amplification), conducted on such a sample. Such assays are well known in the art, as are their comparative merits. Reviews comparing CDC assays against typical SAB-HLA assays (sometimes referred to as Luminex assays) are available in, for example, Tait et al, NEPHROLOGY 2009; 14, 247-254 and Mulley and Kanellis, NEPHROLOGY 2011; 16, 125-133. Solid-phase bead assays are also discussed in Tait, FRONTIERS IN IMMUNOLOGY December 2016; 7, article 570, 1-11. A discussion of the detection of DSA specifically in the context of HSPC transplantation is provided in Ciurea et al, BONE MARROW TRANSPLANT 2018; 53(5): 521-534 (see in particular section 3).

Solid-phase assays are preferred in connection with the methods described herein, with the SAB-HLA type assay particularly preferred. This type of assay is now used in the majority of transplant centres to detect and quantify DSA levels. The assay involves the incubation of a patient sample (usually serum) with fluorescently labeled beads. Each bead has one type of HLA (Human Leukocyte Antigen) molecule attached, and is labelled such that there is a unique signal for each said HLA molecule. If the patient has antibodies against a particular HLA molecule, then those antibodies will react with that population of beads to which that HLA molecule is attached, and can therefore be quantified by detection of the associated signal, typically read out as a mean fluorescence intensity (MFI). Since the types of HLA molecule present in the donor can be known, it is possible to determine whether the patient has donor specific antibody (DSA) and in what quantities they are present. A MFI level >1000 for at least one donor HLA molecule is typically taken to be DSA positive and hence may indicate a sensitized patient. A MFI level >5000 for at least one donor HLA molecule indicates an increased risk of complement-fixation, and thus may be taken to indicate a highly-sensitized patient. A MFI level of >10,000 for at least one donor HLA molecule may indicate a very highly-sensitized patient, such that the patient requires a higher initial dose of IgG inactivating enzyme. For example such a patient may require around double the usual dose as discussed in the section relating to enzymes above.

The inventors have also identified a method to identify sensitized patients who may particularly benefit from the conditioning regimen of the invention. The method identifies such patients based on either very high initial levels of DSA, or on a predicted recovery of DSA to problematic levels within 14 days of a first dose IgG inactivating enzyme. The method relies on the measurement of DSA in samples taken prior to the first dose of IgG inactivating enzyme (i.e. pre-dose), preferably using a SAB-HLA assay to obtain a readout in MFI for at least one HLA molecule present in the donor, essentially as described above. However, the MFI value obtained from the undiluted pre-dose sample is then compared to the MFI value obtained from a 10× diluted pre-dose sample from the same subject assessed in the same assay. In general terms, in patients considered to be sensitive in accordance with standard criteria (MFI level >1000 for at least one donor HLA molecule in an undiluted pre-dose sample), the MFI value in a 10× diluted pre-dose sample has been found to be predictive of the corresponding MFI value that would be obtained if the assay were to be repeated on a sample taken from the same patient at around 14 days post-transplant, if a second dose of IgG inactivating enzyme is not administered in accordance with the conditioning regimen described herein. More specifically, the MFI value in the 10× diluted sample has been observed to be typically within the range of 0.5x to 2.5x the MFI value that is obtained using the same assay on an undiluted sample taken from the same patient at around 14 days post-transplant in the absence of if a second dose of IgG inactivating enzyme. Thus, if the MFI value for at least one donor HLA molecule in the 10× diluted pre-dose sample is >1000 (or preferably >2000), this predicts that the donor-specific response in the patient will have recovered to potentially problematic levels within 14 days and hence that a second dose of IgG inactivating enzyme should be administered in accordance with the conditioning regimen described herein.

In other words, also provided is a method of determining whether a patient is sensitized to a donor and requires the application of the conditioning regimen described herein with any cell transplant from that donor. The method may comprise the following steps:
(i) The DSA level for at least one HLA molecule present in the donor is measured by SAB-HLA assay in an undiluted sample obtained from the patient prior to an initial IgG-inactivating enzyme dose to give a first MFI value;
(ii) The DSA level for the same at least one HLA molecule present in the donor is measured by SAB-HLA assay in a 10× dilution of a sample obtained from the patient prior to an initial IgG-inactivating enzyme dose to give a corresponding second MFI value; and
(iii) The first and second MFI values are used to determine whether or not the patient is sensitized and should be treated with the conditioning regimen, including in particular a second dose of IgG inactivating enzyme, wherein the following criteria are applied:
   (A) First MFI value between 1000 and 10,000 for at least one HLA molecule present in the donor, plus the corresponding second MFI value is reduced by at least 50% relative to the first MFI value and is at a level that a physician considers would be unsafe for cell transplantation (preferably >1000, more preferably >2000), this indicates that the patient is sensitized and the conditioning regimen should be used.
   (B) First MFI value between 1000 and 10,000 for at least one HLA molecule present in the donor, plus the corresponding second MFI value is not reduced by at least 50% relative to the first MFI value, this indicates that the patient is sensitized and the conditioning regimen should be used, optionally including a higher initial dose of IgG-inactivating enzyme;
   (C) First MFI value >10,000 for at least one HLA molecule present in the donor indicates that the patient is sensitized and the conditioning regimen should be used, optionally including a higher initial dose of IgG-inactivating enzyme.

If the criteria of (A) are met, this typically indicates that the patient is likely to experience a rebound of donor-specific responses to problematic levels within 14 days absent a second dose of IgG inactivating enzyme, and thus the conditioning regimen described herein should be used to increase the duration of the period in which DSA levels are low enough to permit successful engraftment of a cell transplant.

If the criteria of (B) or (C) are met, this typically indicates that the initial level of DSA in the patient is very high. A very high initial level is suggestive that donor-specific responses may be problematic relatively early post-transplant (i.e. within 14 days) and so again the conditioning regimen described herein should be used to increase the duration of the period in which DSA levels are low enough to permit successful engraftment of a cell transplant. In addition, the existence of a very high initial level of DSA is the reason that the option of a higher initial dose of IgG-inactivating enzyme is provided.

If a pre-dose sample is not determined to have MFI level >1000 for any HLA molecule present in the donor, then this likely means they are not sensitized to the donor and so the conditioning regimen described herein may not be required.

The patient determination method, or any other method involving use of a SAB-HLA assay, may optionally include an additional step to mitigate the prozone effect. Such a step will typically comprise pre-treatment of the sample before conducting the assay, for example by the addition of dithiothreitol and/or EDTA and/or heat-treatment. Mitigation of the prozone effect may be conducted in accordance with standard procedures known in the art, as discussed in the section "The Prozone Effect" in Tait, FRONTIERS IN IMMUNOLOGY December 2016; 7, article 570, 1-11.

### Conditioning regime - additional steps or agents

The conditioning regimen may additionally comprise one or more of:
- a suitable interval before step b (transplant), typically 1 day, administering to the subject a non-lethal dose of irradiation and/or any other agent which depletes the subject's HSPC, for example Total Body Irradiation (TBI) at 4 Gy;
- a suitable interval after step c (second enzyme dose), typically at least 48 hours, administering to the subject an agent which reduces the risk of infection caused by low IgG levels, such as intravenous gamma globulin (IVIG);
- a suitable interval before step b (transplant), typically at least 1 day, administering to the subject any other agent or regimen which modulates (e.g. reduces) the activity of the immune system, including inhibitors of complement, inhibitors of cytokines, inhibitors of innate immune cells, inhibitors of plasma cells, inhibitors of lymphocytes, inducers of tolerance. This step may be conducted by any suitable method and using any suitable agent.

The same agent or combination of agents may be effective to reduce the numbers and/or down-modulate the activity of more than one type of lymphocyte. This may be referred to as lymphocyte depletion or lymphodepletion. The agents may be referred to as lymphodepleting agents. The agents are preferably administered at a dose sufficient to substantially reduce the numbers and/or substantially down-modulate the activity of lymphocytes in the subject. Preferred agents target T lymphocytes and/or B lymphocytes, with T lymphocytes most preferred. The agent may also deplete NK cells and/or non-malignant hematopoietic disease precursors.

Exemplary agents suitable for the depletion of lymphocytes (which may be referred to as lymphodepleting agents) are known in the art and include:
- An anti-CD52 antibody, such as alemtuzumab (also known as Campath-Hl). Alemtuzumab is a humanized monoclonal antibody against CD52 and causes depletion of T and B lymphocytes, NK cells, monocytes, granulocytes, and dendritic cells, which all express CD52. Three mechanisms contribute to the depletion of these cells by alemtuzumab: CDC, ADCC and apoptosis. The half-life of alemtuzumab is generally reported at 12 days (288h), but in practice it depends on the concentration of its target. The plasma half-life of alemtuzumab is shorter (around 6 days) in patients with CLL with a large tumor burden due to cytotoxicity of malignant cells and clearance from the blood. In contrast, after successful treatment of a CLL patients, when CD52 levels decrease, the half-life of alemtuzumab increases to 8-21 days. Maintenance of a therapeutic concentration of 100 ng/ml for 56 days after HSPC transplant has been reported. No antidote to accidental overdose of alemtuzumab is known and it is unlikely that alemtuzumab would be removed with hemodialysis due to its size (150 kDa). As such there is currently no effective way to reduce the concentration of alemtuzumab in a patient (such as HSPC transplant recipient) to reduce the risk of relapse and infection. An enzyme as disclosed herein which inactivates serum IgG molecules can be used to achieve this goal, in particular an IgG cysteine protease enzyme as discussed below, preferably imlifidase. Thus, also disclosed herein is the treatment of alemtuzumab overdose by administration of such an enzyme, preferably imlifidase.
- A polyclonal mixture of non-human antibodies directed against human lymphocyte antigens, typically human T and/or B lymphocyte antigens. Various mixtures are well known in the art, and may commonly be referred to by the general term ATG, and specifically rATG (for rabbit originating mixtures) or hATG (for horse originating mixtures). The term "ATG" is taken from the International Non-Proprietary Names for such mixtures, which include (rabbit) anti-human T-lymphocyte immunoglobulin, or (rabbit) anti-human thymocyte globulin. Different "ATG" mixtures will have different antibody profiles and so may have differences in their precise immunosuppressive activities. However, all are a mixture of antibodies intended to bind to various T and B cell antigens, leading to T- and B lymphocyte depletion via (i) apoptosis via activation-induced cell death, (ii) antibody-dependent cell-mediated cytotoxicity, and (iii) complement-dependent cytotoxicity. Currently there are two ATG preparations available for use as lymphodepleting agents in human patients. Both are prepared in rabbits and so may be interchangeably referred to herein by the general term rATG, unless a specific mixture is intended, in which case this will be specified. The two mixtures differ in terms of the immunogen used to raise them and as a consequence each has different antigen specificities. The first mixture may be referred to as ATG-F (for Fresenius) or ATG-G (for Grafalon) and is produced by immunizing rabbits with the T-cell leukemia line Jurkat. The antibody profile in this mixture is rarely against CD3, CD4, CD44, and HLA-DR but targeting CD28, CD29, CD45, CD49, CD98, CD147, and more antibodies directed against CD107 (an antigen expressed on T cells during degranulation following antigenic stimulation). It has a reported half-life of 2-3 days but can be measured in the recipients' blood for up to 5 weeks after infusion. The second mixture may be referred to as Thymoglobulin and is produced by immunizing rabbits with fresh human thymocytes. The antibody profile in this mixture is typically against CD2, CD3, CD4, CD8, CD11a, CD18, CD25, CD44, CD45, HLA.DR, HLA Class I heavy chains and b2-microglobulin. It has a reported half-life of 30 days, with a total dose of 10 mg/kg for 4 days (at Days -4 to - 1) detectable for 8 weeks after infusion and active agent still detectable at Day +28.
   - Other agents may include any one or more of:
      *Other agents primarily depleting T cells*
         - a panel of antibodies including anti-CD4, anti-CD8, and anti-CD90; or
         - an anti-CD117 antibody; or
         - an anti-CD47 antibody; or
         - an anti-CD45 antibody; or
         - an anti-CD38 antibody (for example daratumumab); or
         - abatacept; or
         - an immunotoxin targeting T cells; or
      *Other agents primarily depleting B cells (optionally including plasma cells)*
         - an anti-CD20 antibody (such as rituximab);
         - an anti-CD 19 antibody;
         - an immunotoxin targeting B cells, such as an anti-CD20 immunotoxin (for example MT-3724).

The conditioning regimen may additionally include administration also of a non-IgG based agent that is suitable to reduce the numbers and/or down-modulates the activity of lymphocytes in the subject. For example, any one or more of busulfan, cyclophosphamide, fludarabine, treosulfan, cyclosporine, bortezomib and tacrolimus may be used to deplete or inactivate T and/or B cells.

### Method for prevention or treatment of immune rejection of a cell transplant

The invention provides a method for the prevention or treatment of immune rejection of a cell transplant to a sensitized subject, the method comprising administering a cell transplant to the subject in accordance with the conditioning regimen of the invention. The method may also be described as a method for the prevention or treatment of engraftment failure or poor graft function of a cell transplant to a sensitized subject, the method comprising administering a cell transplant to the subject in accordance with the conditioning regimen of the invention.

The invention also provides an enzyme which inactivates serum IgG molecules in a subject for use in a method for the prevention or treatment of immune rejection, engraftment failure or poor graft function, of a cell transplant to a sensitized subject, the method comprising administering a cell transplant to the subject in accordance with the conditioning regimen of the invention.

The invention also provides the use of an enzyme which inactivates serum IgG molecules in a subject in the manufacture of a medicament, wherein the medicament is for use in a method for the prevention or treatment of immune rejection, engraftment failure or poor graft function, of a cell transplant to a sensitized subject, the method comprising administering a cell transplant to the subject in accordance with the conditioning regimen of the invention.

The method may alternatively be described as a method for the induction of hematopoietic chimerism in a subject, the method comprising conducting the conditioning regimen of the invention and subsequently administering HSPC to the subject in an amount sufficient and under conditions suitable to induce hematopoietic chimerism in the subject. The method may alternatively be described as a method for the stable transplantation of HSPC. In all cases, the HSPC may be autologous (the patient's own cells are used) or syngeneic (the cells are from a genetically identical twin), or they may allogeneic (the cells come from a separate, non-identical donor).

Immune complications which reduce the likelihood of successful engraftment of HSPC in the recipient are most significant for allogeneic cells and thus the method of the invention is of greatest benefit with such cells. However, immune complications can occur even with autologous cells if there is expression of a product to which the recipient has not previously been exposed. If an autologous cell has been genetically modified to express a gene therapy, the cell may be sufficiently altered to provoke an immune response. For example there may be an immune response to the expressed gene therapy product. Similar would apply if the HSPC has been genetically modified to express a different HLA type which is not matched to the HLA of the recipient. Therefore the HSPC are preferably allogeneic, or are genetically modified autologous or syngeneic cells, for example Chimeric antigen receptor (CAR) T-cells. The HSPC are most preferably allogeneic. In a particularly preferred embodiment, the HSPC are from a donor who is also the donor of another organ or tissue which is to be transplanted into the recipient. That is, the same donor provides both the HSPC and the other cell, organ or tissue.

HSPC are found in the bone marrow of adults, especially in the pelvis, femur, and sternum. They are also found in umbilical cord blood and, in small numbers, in peripheral blood. HSPC may be harvested from these locations using any suitable technique established in the art.

For example, HSPC may be harvested from human bone marrow by aspirating directly from the centre of a bone of the donor with a large needle. The posterior iliac crest is the usual site of harvest. The technique is referred to as a bone marrow harvest and may be performed under local or general anesthesia. When the administered HSPC are derived from the bone marrow of the donor, the administration of HSPC may be described as a bone marrow transplant (BMT).

HSPC may be harvested from umbilical cord blood shortly after the birth of an infant. The umbilical cord is double-clamped from the umbilicus and transacted between clamps. The umbilical cord vein is then punctured under sterile conditions, and the blood flows freely by gravity into an anticoagulated sterile closed harvesting system, form which the HSPC may be isolated.

HSPC may be harvested from peripheral blood, typically by apheresis. However, because numbers of HSPC in peripheral blood are normally low, it is first necessary to mobilize HSPCs from the bone marrow. In a healthy donor, this can be achieved by administration of Granulocyte colony-stimulating factor (G-CSF). Alternative strategies may be required if the donor is not healthy. This may frequently be the case if the intended HSPC transplant is autologous.

HSPC are preferably used as quickly as possible after harvesting (that is fresh), but may be cryopreserved for storage prior to thawing for use in the method of the invention. Cryopreservation typically includes volume depletion by removal of red cells and plasma. The quantity of stem cells in the harvest may be quantified, e.g. by flow cytometric analysis of a sample, to establish the proportion of cells which are positive for CD34 (a marker for stem cells).

The HSPC may be administered to the subject by any suitable method. A preferred method is infusion, typically through a central line. The patient may be kept in highly clean or sterile conditions, such as in a room with high-efficiency particulate air (HEPA) filters under positive pressure, before, during and after the infusion to reduce the risk of infection.

The method may be monitored to determine that the HSPC transplant has successfully resulted in hematopoietic chimerism. This is achieved by determining the proportion of donor-derived hematopoietic cells present in a blood sample taken from the subject after a particular time interval, typically 28 days after administration of the HSPC. For example, hematopoietic chimerism may be defined as achieved if at least 5% of the lymphocytes and/or myeloid cells in the sample are found to be donor-derived, preferably if at least 5% of the lymphocytes in the sample are found to be donor-derived. The chimerism is described as mixed if no more than 90% of the lymphocytes and/or myeloid cells in the sample are found to be donor-derived (that is at least 10% are still derived from the recipient), preferably if no more than 90% of the lymphocytes in the sample are found to be donor-derived (that is at least 10% of lymphocytes are still derived from the recipient). The chimerism may be described as total if 98% or more of the lymphocytes and/or myeloid cells in the sample are found to be donor-derived. Mixed chimerism is typically preferred for the methods of the invention, because the recipient will have a greater level of immunocompetence. However, full chimerism may be beneficial in some circumstances, for example in the treatment of cancers such as leukemia where the goal is to eliminate host cells with the potential to cause cancer recurrence, replacing them with the transplanted HSPC.

The proportion of donor and recipient derived cells in a sample may be determined by any suitable method in the art, such as flow cytometric analysis as described in the Examples. Real-time PCR may also be used. Other methods are discussed in Agrawal et al Bone Marrow Transplantation 2004 (34) p-12.

### Methods of treating or preventing a disease or condition

The present invention provides a method for the prevention or treatment of a disease or condition in a subject. The method comprises administering a cell transplant to the subject, wherein said administering comprises the conditioning regimen of the invention. Expressed another way, the invention also provides a method for the prevention or treatment of immune rejection of a cell transplant, the method comprising the conditioning regimen of the invention.

The cell transplanted may be of any type, including HSPC. The HSPC may be a genetically modified HSPC, such that the method effectively comprises administering a gene therapy to the subject in which the HSPC is the vector. The cell to be transplanted may originate from a different species to the recipient, that is it may be a xenotransplant. Suitable species for xenotransplantation into human recipients may include pigs or non-human primates. In such cases the HSPC may be genetically modified to aid with tolerance to the transplant. The cell that is a xenotransplant may also be genetically modified.

The subject to be treated may be sensitized or highly sensitized. By "sensitized" it is meant that the subject has developed antibodies to human major histocompatibility (MHC) antigens (also referred to as human leukocyte antigens (HLA)). The anti-HLA antibodies originate from allogeneically sensitized B-cells and are usually present in patients that have previously been sensitized by blood transfusion, previous transplantation or pregnancy. Achieving hematopoietic chimerism in sensitized patients may reverse allosensitization, through the generation of specific tolerance in T and B cells, resulting in a reduction of donor specific immune responses such as DSA.

Whether or not a potential transplant recipient is sensitized may be determined by any suitable method. For example, a Panel Reactive Antibody (PRA) test may be used to determine if a recipient is sensitized. A PRA score >30% is typically taken to mean that the patient is "high immunologic risk" or "sensitized". Alternatively, a cross match test may be conducted, in which a sample of the potential transplant donor's blood is mixed with that of the intended recipient. A positive cross-match means that the recipient has antibodies which react to the donor sample, indicating that the recipient is sensitized and transplantation should not occur. Cross-match tests are typically conducted as a final check immediately prior to transplantation.

The method may be for the prevention or treatment of any disease or condition that is treated by HSPC transplant. Diseases or conditions typically treated by HSPC transplant may be acquired or congenital. Acquired diseases or conditions that may be treated by HSPC transplant include:
- Hematological malignancies such as leukemias (for example Acute lymphoblastic leukemia (ALL), Acute myeloid leukemia (AML), Chronic lymphocytic leukemia (CLL), Chronic myelogenous leukemia (CML); lymphomas (for example Hodgkin's disease, Non-Hodgkin's lymphoma) and Myelomas (for example, Multiple myeloma (Kahler's disease));
- Hematologic diseases, such as phagocyte disorders (for example Myelodysplasia) or Hemoglobinopathies (for example Sickle cell disease, β thalassemia major (Cooley's anemia);
- Anemias (for example Paroxysmal nocturnal hemoglobinuria (PNH; severe aplasia), Aplastic anemia, Acquired pure red cell aplasia, Diamond-Blackfan anemia, Fanconi anemia); Hemoglobinopathies (for example Sickle cell disease, β thalassemia major (Cooley's anemia), Cytopenias (for example Amegakaryocytic thrombocytopenia); and Hemophagocytic syndromes (for example Hemophagocytic lymphohistiocytosis (HLH);

- Myeloproliferative disorders (for example Polycythemia vera, Essential thrombocytosis, Myelofibrosis);
- Solid tumor cancers, such as Neuroblastoma, Desmoplastic small round cell tumor, Ewing's sarcoma, Choriocarcinoma;
- Metabolic disorders such as amyloidosis (for example Amyloid light chain (AL) amyloidosis);
- Environmentally-induced diseases such as radiation poisoning;
- Viral diseases such as Human T-lymphotropic virus (HTLV) or Human Immunodeficiency Viruses (HIV);
- Autoimmune diseases, such as multiple sclerosis;
   Lysosomal storage disorders such as Lipidoses - disorders of lipid storage - (for example Neuronal ceroid lipofuscinoses, Infantile neuronal ceroid lipofuscinosis (INCL, Santavuori disease,), Jansky-Bielschowsky disease (late infantile neuronal ceroid lipofuscinosis)); Sphingolipidoses (for example Niemann-Pick disease, Gaucher disease); Leukodystrophies (for example Adrenoleukodystrophy, Metachromatic leukodystrophy, Krabbe disease globoid cell leukodystrophy)); Mucopolysaccharidoses (for example Hurler syndrome (MPS I H, α-L-iduronidase deficiency), Scheie syndrome (MPS I S), Hurler-Scheie syndrome (MPS I H-S), Hunter syndrome (MPS II, iduronidase sulfate deficiency), Sanfilippo syndrome (MPS III), Morquio syndrome (MPS IV), Maroteaux-Lamy syndrome (MPS VI), Sly syndrome (MPS VII)); Glycoproteinoses (for example Mucolipidosis II (I-cell disease), Fucosidosis, Aspartylglucosaminuria, Alpha-mannosidosis; or Others (for example Wolman disease (acid lipase deficiency);
- Immunodeficiencies, such as T-cell deficiencies (for example Ataxia-telangiectasia, DiGeorge syndrome); Combined T- and B-cell deficiencies (for example Severe combined immunodeficiency (SCID), all types); Wiskott-Aldrich syndrome; Phagocyte disorders (for example Kostmann syndrome, Shwachman-Diamond syndrome); Immune dysregulation diseases (for example Griscelli syndrome, type II); Innate immune deficiencies (for example NF-Kappa-B Essential Modulator (NEMO) deficiency.

Where the HSPC are genetically modified to administer a gene therapy, the method of the invention may be for the prevention or treatment of the disease or condition to which said gene therapy is directed.

The invention also provides an enzyme which inactivates serum IgG molecules in a subject for use in a method for the prevention or treatment of a disease or condition, wherein the method is as described above.

The invention also provides the use of an enzyme which inactivates serum IgG molecules in a subject in the manufacture of a medicament, wherein the medicament is for the prevention or treatment of a disease or condition in a method as described above.

### Production of polypeptides

The enzymes used in the methods of the invention are polypeptides and may be produced by any suitable means. For example, a polypeptide may be synthesised directly using standard techniques known in the art, such as Fmoc solid phase chemistry, Boc solid phase chemistry or by solution phase peptide synthesis. Alternatively, a polypeptide may be produced by transforming a cell, typically a bacterial cell, with a nucleic acid molecule or vector which encodes said polypeptide. Production of enzyme polypeptides by expression in bacterial host cells is described and exemplified in WO2016/128558 and WO2016/128559.

### Compositions and formulations comprising polypeptides

The present invention also provides compositions comprising an enzyme for use in the methods of the invention. For example, the invention provides a composition comprising one or more polypeptides, and at least one pharmaceutically acceptable carrier or diluent. The carrier(s) must be 'acceptable' in the sense of being compatible with the other ingredients of the composition and not deleterious to a subject to which the composition is administered. Typically, carriers and the final composition are sterile and pyrogen free.

Formulation of a suitable composition can be carried out using standard pharmaceutical formulation chemistries and methodologies all of which are readily available to the reasonably skilled artisan. For example, the enzyme can be combined with one or more pharmaceutically acceptable excipients or vehicles. Auxiliary substances, such as wetting or emulsifying agents, pH buffering substances, reducing agents and the like, may be present in the excipient or vehicle. Suitable reducing agents include cysteine, thioglycerol, thioredoxin, glutathione and the like. Excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethylene glycol, hyaluronic acid, glycerol, thioglycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

Such compositions may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition for parenteral administration, the active ingredient is provided in dry (for e.g., a powder or granules) form for reconstitution with a suitable vehicle (e. g., sterile pyrogen-free water) prior to parenteral administration of the reconstituted composition. The compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono-or di-glycerides.

Other parenterally-administrable compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. The compositions may be suitable for administration by any suitable route including, for example, intradermal, subcutaneous, percutaneous, intramuscular, intra-arterial, intraperitoneal, intraarticular, intraosseous or other appropriate administration routes. Preferred compositions are suitable for administration by intravenous infusion.

### Kits

The invention also provides a kit for carrying out the methods described herein. The kit of the invention may include an enzyme or a composition comprising an enzyme, as described above. The kit may include means for administering the enzyme or composition to a subject. The kit may include instructions for use of the various components in any method as described herein.

### EXAMPLES

Unless indicated otherwise, the methods used are standard biochemistry and molecular biology techniques. Examples of suitable methodology textbooks include Sambrook et al., Molecular Cloning, A Laboratory Manual (1989) and Ausubel et al., Current Protocols in Molecular Biology (1995), John Wiley and Sons, Inc.

### Example 1 - development of a conditioning regimen protocol for desensitization of allosensitized HSPC

### Materials and methods

### Serum samples

Serum samples were collected in clinical studies, predose and at different timepoints post imlifidase dosing and SAB-HLA assay was run within the clinical studies (EudraCT Numbers 2014-000712-34 and 2016-002064-13). For the in vitro enzyme treatments, predose samples from anonymized samples in the same studies were used.

### SAB-HLA

Serum samples were pretreated with 5 mM EDTA to reduce the prozone effect. LABScreen beads (cat no. LS1A04 or LS2A01 for class I and II respectively, both from OneLambda) were incubated with serum (5 µL beads + 20 µL serum) for 30 min in the dark at room temperature with gentle shaking. After incubation with serum, the beads were washed four times with 1× wash buffer (diluted in water from cat no LSPWABUF, OneLambda) and incubated with 1× PE-conjugated anti-human IgG (diluted in 1× wash buffer from cat no LS-AB2, OneLambda) for 30 min in the dark at room temperature with gentle shaking. The beads were washed twice as above, resuspended in 80 µL phosphate buffered saline and data was acquired using a Luminex 100 machine.

### Results and discussion

To develop a treatment protocol for successful desensitization of allosensitized HSPC transplant patients, we studied the DSA rebound patterns after imlifidase treatment in sensitized transplant patients to identify when in time a potential second dose of an IgG-cleaving enzyme is needed post-transplant to ensure that circulating stem cells are protected from harmful DSAs.

The mean DSA-rebound pattern in a study of sensitized transplant patients is shown in Fig 1. The patients were classified as DSA positive based on a DSA level measured by SAB-HLA assay of at least 1000 MFI (mean fluorescent intensity). All DSAs are cleaved and reach well below 1000 MFI early after dosing with IgG cleaving enzyme (imlifidase) and, furthermore, DSA levels remain low (below 5000 MFI) without further treatment. Other published data is in line with these findings. However, from closer examination of the data for individual subjects it is clear that there is a large individual variation in levels and rebound times. In Fig. 2 this is exemplified by three individual patients from the same study, where HLA class I and II MFI levels are shown. It is clear that the individual variation of rebounding HLA levels is large and despite this it has been shown that a single dose of imlifidase prior to kidney transplant is efficient and allow for successful transplantation. As stem cells are expected to be more sensitive to rebounding antibodies, levels that could be problematic for cell transplants are likely to be reached sooner in these patients.

In the present study, 59 DSAs were found to be above 1000 MFI in samples taken before enzyme administration (pre-dose), and were therefore assigned as DSA positive, suggesting potential sensitivity. The individual data further show that 23 out of 59 DSAs had rebounded to above 1000 MFI at day 7 (39%), 18 out of 59 to above 2000 MFI (31%) and 13 out of 59 to above 5000 at day 7 (22%). As rebound of DSA has to be minimized to allow for successful transplant of HSPC and it is not yet known what level of MFI in SAB-HLA assays represents a safe DSA level to reach engraftment, this suggests that (unlike in a solid organ transplant) a second dose should be included prior to day 7 and certainly prior to day 14, to increase the DSA-free window in all patients. In addition, certain patients in the study had a very high pre-dose level of DSA, with MFI level >10,000. In these patients, the rebound of DSA is readily apparent even at 5 days post-dosing - see Fig. 7.

Overall, to reduce the likelihood of DSA rebound to levels that will jeopardize engraftment, a conditioning regimen for HSPC transplant should include administration of a first dose of IgG cleaving enzyme prior to cell infusion (typically not earlier than 24 hours and not later than 4 hours prior), and a second dose of IgG cleaving enzyme at up to 14 days after the first enzyme dose, preferably between 3 and 7 days, and most preferably at around 5 days after the first enzyme dose.

### Example 2 - use of pre-dose samples to predict sensitized patients who will benefit from the enhanced regimen

Consistent with the finding in Fig. 2, several patients in kidney transplant studies have required treatment to intervene with a suspected antibody-mediated rejection (AMR) episode, despite earlier treatment with IgG cleaving enzyme. This was monitored by DSA rebound pattern (using SAB-HLA) but critically also using kidney function tests and biopsies to evaluate the suspected acute AMR. For HSPC transplant, there is no functional readout that can be used to monitor the cells prior to successful engraftment, and bone marrow biopsies at early time-points are not informative. This means the only possible monitoring at present is via the DSA rebound pattern (e.g. by SAB-HLA). Even this may be problematic, because in some kidney transplant patients it was found that post-transplant DSA levels did not correlate with biopsy findings. An example is shown in Fig. 3. DSA rebound in this patient started at day 2-3 and continued rising rapidly over the coming days. However, no AMRs were reported during the study for this patient, and the day 180 biopsy showed no signs of AMR (g0, ptc0, C4d3 despite the high MFI levels). Thus, only monitoring SAB-HLA may not be informative for outcome.

We looked at different ways of analyzing samples and found that 10× diluted samples taken before the IgG cleaving enzyme is administered (i.e. pre-dose) can provide a prediction of the expected DSA level at day 14 after enzyme treatment (i.e. post-dose), when DSA level is given as the MFI level in a SAB-HLA assay. Specifically, when DSA is measured by SAB-HLA to give a readout in MFI level in a sample taken pre-dose, and when this is compared to DSA measured by SAB-HLA to give a readout in MFI level in a 10× diluted portion of a sample taken pre-dose, this comparison can be used to predict the expected MFI at day 14 post-dose and hence to identify patients for whom the conditioning regimen of the present invention will be particularly beneficial. The steps of the resulting patient identification methods are described in the detailed description above.

In the present study, 59 DSAs were found to be above 1000 MFI in the pre-dose sample and were therefore assigned as DSA positive. Provided that the corresponding MFI level in the 10× pre-dose sample was reduced by at least 50% relative to the undiluted sample, then the MFI level in the 10× pre-dose sample was predictive of the corresponding MFI level in samples obtained 14 days post-transplant in 92% of cases (54 of 59 DSAs). This is illustrated in Figs 4 and 5.

In Fig. 4 three patients with one DSA above 1000 at predose (that is, an MFI level >1000 for at least one HLA molecule present in the donor) are exemplified showing the 10× dilution (Pre a 10×) run in the same experiment as the predose sample (Pre a) and compared to a predose sample as well as samples collected 24 hours, 7 days and 14 days post imlifidase treatment (the latter four samples run in separate experiment). The figure shows that the day 14 sample is in the same range as the 10× diluted sample.

In Fig. 5 three patients with several DSAs above 1000 at predose (that is, an MFI level >1000 for multiple types of HLA molecule present in the donor) are exemplified showing the 10× dilution (Pre a 10×) run in the same experiment as the predose sample (Pre a) and compared to the predose sample as well as samples collected 24 hours, 7 days and 14 days post imlifidase treatment (the latter four samples run in separate experiment). The figure shows that the day 14 sample is in the same range as the 10× diluted sample.

### Example 3 - use of pre-dose samples to predict very highly-sensitized patients who may additionally require higher doses of enzyme

Again working with 10× diluted pre-dose samples as in Example 2, some patients were found in whom the dilution did not lower the DSA reading by at least 50% relative to the undiluted sample. The inventors believe that this suggests that the MFI levels in these subjects are so high that it may be necessary to increase the initial dose of IgG cleaving enzyme. The existence of such patients is also evidence from Fig. 7. The initial dose could be increased by giving a higher single dose, or by administering the same initial dose two or more times in a 24 hour interval. For example, the typical recommended dose of imlifidase before solid organ transplant is around 0.25 mg/kg BW. This could be doubled to around 0.5 mg/kg BW, administered as one dose of 0.5 mg/kg BW, or as two doses of 0.25 mg/kg BW within 24 hours. This adjustment is based on the expected sensitivity of HSPC vs kidney cells and is supported by data from patients in the same study as Examples 1 and 2, who exhibited DSA levels that were not decreased by at least 50% in the 10× diluted samples. This is exemplified in Fig. 6 showing the patients for whom the 10× dilution did not show at least 50% reduction in MFI relative to undiluted, as well as their actual MFI level 24 hours post dosing.

### Example 4 - kinetics of IgG-cleaving enzymes further supports a second dose

Published data with imlifidase show that a single dose of enzyme can efficiently inactivate intra- and extravascular IgG and flow back from extravascular space is slow and in the form of scIgG. However, there is a constant production of IgG in all humans at a rate generating a steady state of around 10 mg/mL IgG and this production is needed to maintain steady state as the half-life of IgG is in the range of 7 days (IgG3) to 21 days (IgG1, IgG2 and IgG4). Despite the ongoing production of IgG, intact IgG is not seen in serum for 1-2 weeks post-imlifidase administration (Winstedt PLOS 2015 and Jordan NEJM 2017). This was surprising and prompted further investigation. Imlifidase has a short distribution half-life of a few hours and a longer elimination half-life of several days, but as the concentration drops very fast to levels that are not sufficient to cleave IgG in humans, remaining imlifidase concentrations were not expected to contribute to the degradation of IgG. As there was a large individual variation in the IgG rebound pattern in the clinical studies and there was no correlation between this pattern and the individual PK profile, this prompted further investigation.

Titration experiments in sera showed that a concentration of around 2 µg/mL imlifidase was needed to start the cleavage in serum and concentrations below this did not result in any cleavage despite prolonged incubation. A serum pool (n = 100) was treated with 0.5, 1, 2 or 4 µg/mL imlifidase for 1 to 48 hours and analysed by SDS-PAGE (see Fig. 8). It is demonstrated that a concentration of around 2 µg/mL imlifidase was needed to start the cleavage in serum and concentrations below this did not result in any cleavage despite prolonged incubation. Other data (not shown) make it clear that in patients approximately 24 hours after dosing there is around 2 µg/mL imlifidase (or less) in circulation and 48 h after dosing there was less than 1 µg/mL left. Such concentrations were not expected to contribute to IgG degradation in humans. To confirm this we spiked in two different substrates in the sera collected at different time-points and surprisingly found that there was imlifidase activity left in the samples for a substantially longer period, and with a huge difference in substrate-degradation depending on the substrate added. When polyclonal IgG was used as substrate, no degradation was seen in samples collected from 48 hours after dosing and forward, but when IgG1 was used as a substrate there was continuous conversion of intact IgG to scIgG even at the 7-day timepoint. These surprising findings suggest that despite very low remaining imlifidase concentrations present beyond 2-3 day from dosing, those concentrations may still be enough to cleave the newly produced IgG into scIgG. This confirms that a second dose at around 3-7, preferably 5 days, after the first dose of IgG-cleaving enzyme is likely to further expand the window where there is no intact IgG, mitigate for the large individual variation in rebound and reduce the risk of graft failure.

### Example 5 - exemplary conditioning regimen

Patients in need of desensitization prior to transplant of HSPC (abbreviated as HSCT) will be treated in accordance with the protocol shown in Fig. 9, which exemplifies the present invention.

Patients in need of HSCT who have donor-specific antibodies against their potential donor can be desensitized using the protocol outlined in Fig. 9. The level of sensitization to the available donor can be appreciated using the SAB-HLA assay conducted with neat serum and 10× diluted serum as discussed in the preceding Examples.

If intravenous gamma globulin (IVIg) has been used as a desensitization strategy prior to deciding on using an IgG-degrading enzyme for the patient, it is important to allow a sufficient time before enzyme administration to make sure that anti-drug antibodies present in IVIg do not impact the safety and efficacy of the enzyme. The recommended time to wait is presently 28 days.

The conditioning regimen may be dependent on what is standard of care (SoC) for the specific indication prior to HSCT at each specific treatment location. For instance, SoC is often a combination of chemotherapy and radiotherapy starting 1-2 weeks prior to HSCT. Premedication with corticosteroids and anti-histamines may be given prior to enzyme administration. The first dose of IgG-degrading enzyme should be administered 4-24 hours prior to the HSCT. The enzyme dose is typically either 0.25 mg/kg, 0.5 mg/kg or 2x 0.25 mg/kg. If the 2x 0.25 mg/kg dose is used, both should be administered prior to HSCT and not separated by more than 24 hours. If the 10× diluted sample test performed prior to enzyme dosing indicate that the donor specific antibodies are very high (not decrease by at least 50% vs neat) the 0.5 mg/kg dose should be considered.

After HSCT a graft vs host disease (GvHD) prophylaxis may be needed, and this can be any type of prophylaxis that is SoC after HSCT at the treatment location. Preferably, the prophylaxis is based on post-transplant cyclophosphamide (PTCy), mycophenolate mofetil (MMF) and tacrolimus. GvHD prophylaxis based on antibodies cannot be used.

A second dose of an IgG-degrading enzyme (typically 0.25 mg/kg) is administered approximately 3-7 days, preferably 5 days, after HSCT followed by IVIg approximately 9 days after HSCT. The earliest timepoint where IVIg can be administered is 48 hours after the last enzyme dose.

## Claims

1. A conditioning regimen for a cell transplant to a sensitized subject, comprising:
a. administering to the subject a dose of an enzyme which inactivates serum IgG molecules in the subject;
b. subsequently administering the transplant to the subject;
c. subsequently administering to the subject a further dose of an enzyme which inactivates serum IgG molecules in the subject, which is optionally the same enzyme as in step a.

2. The conditioning regimen of claim 1, wherein the transplant is of hematopoietic stem and progenitor cells (HSPC), which are optionally:
- autologous or allogenic; and/or
- genetically modified; and/or
- depleted of alpha/beta T-cells.

3. The conditioning regimen of any one of the preceding claims, wherein the sensitized subject has donor-specific antibodies against the available donor.

4. The conditioning regimen of any one of the preceding claims wherein:
- step a. is conducted between about 4 and about 24 hours, preferably about 4 to 6 hours, prior to step b.
- step c. is conducted between about 72 hours and about 336 hours (or between about 3 days and about 14 days), preferably between about 72 hours and about 168 hours (or between about 3 and about 7 days), and more preferably between about 96 hours and about 144 hours (or between about 4 and about 6 days) after step a.

5. The conditioning regimen of any one of the preceding claims, wherein said dose of enzyme administered in step a. and step c. is sufficient to inactivate all or substantially all IgG molecules present in the serum of the subject, optionally wherein the enzyme is an IgG cysteine protease or an IgG endoglycosidase.

6. The conditioning regimen of claim 5, wherein:
(i) the IgG cysteine protease is from a Streptococcus bacterium, such as Streptococcus pyogenes Streptococcus equi or Streptococcus zooepidemicus, optionally wherein said enzyme is a IdeS, MAC2, SpeB, IdeZ or IgdE polypeptide, or
(ii) the IgG endoglycosidase is from a Streptococcus bacterium, such as Streptococcus pyogenes, Streptococcus equi or Streptococcus zooepidemicus, or from Corynebacterium pseudotuberculosis, Enterococcus faecalis, or Elizabethkingia meningoseptica, optionally wherein said enzyme is a EndoS, CP40, EndoE, or EndoF2 polypeptide.

7. The conditioning regimen of claim 5 or 6, wherein:
- said IgG cysteine protease is a polypeptide comprising or consisting of a sequence that is at least 80% identical to SEQ ID NO: 2, 4, 5 such as at least 85%, 90%, 95%, 99% or 100% identical, or wherein said IgG cysteine protease comprises or consists of the sequence of any one of SEQ ID NOs: 6 to 25 and 55 to 69, 91 or 92, optionally wherein said sequence includes an additional methionine at the N terminus and/or a histidine tag at the C terminus; or
- said IgG endoglycosidase is a polypeptide comprising or consisting of a sequence that is at least 80% identical to SEQ ID NO: 90, such as at least 85%, 90%, 95%, 99% or 100% identical, optionally wherein said sequence includes an additional methionine at the N terminus and/or a histidine tag at the C terminus.

8. The conditioning regimen of any one of the preceding claims wherein the enzyme is imlifidase and/or EndoS.

9. The conditioning regimen of any one of the preceding claims wherein the dose of enzyme is between 0.25 mg/kg BW and 0.5 mg/kg BW, preferably between 0.3 mg/kg BW and 0.5 mg/kg BW.

10. The conditioning regimen of any one of the preceding claims, which additionally comprises one or more of:
(a) a suitable interval before step b, typically 1 day, administering to the subject a non-lethal dose of irradiation and/or any other agent which depletes the subject's HSPC, for example Total Body Irradiation (TBI) at 4 Gy;
(b) a suitable interval after step c, typically at least 48 hours, administering to the subject an agent which reduces the risk of infection caused by low IgG levels, such as intravenous gamma globulin (IVIG);
(c) a suitable interval before step b, typically at least 1 day, administering to the subject any other agent or regimen which modulates (e.g. reduces) the activity of the immune system, including inhibitors of complement, inhibitors of cytokines, inhibitors of innate immune cells, inhibitors of plasma cells, inducers of tolerance.

11. The conditioning regimen of claim 10, wherein said agent in (c) is preferably selected from fludarabine, busulfan, melphalan, treosulfan, tacrolimus, cyclophosphamide, cyclosporine, a rabbit antithymocyte globulin (rATG, e.g. ATG-G or Thymoglobulin), an anti-CD52 monoclonal antibody (e.g. alemtuzumab), or any combination thereof.

12. The conditioning regimen of any one of the preceding claims, wherein the patient is determined to be sensitized and to require the application of the conditioning regimen if the level of DSA for at least one HLA molecule present in the donor is determined by SAB-HLA assay in a sample obtained from the patient to have an MFI value >1000; and optionally wherein:
(i) The DSA level for at least one HLA molecule present in the donor is measured by SAB-HLA assay in an undiluted sample obtained from the patient prior to an initial IgG-inactivating enzyme dose to give a first MFI value;
(ii) The DSA level for the same at least one HLA molecule present in the donor is measured by SAB-HLA assay in a 10× dilution of a sample obtained from the patient prior to an initial IgG-inactivating enzyme dose to give a corresponding second MFI value; and
(iii) The first and second MFI values are used to determine whether or not the patient is sensitized and should be treated with the conditioning regimen, wherein the following criteria are applied:
(A) First MFI value between 1000 and 10,000 for at least one HLA molecule present in the donor, plus the corresponding second MFI value is reduced by at least 50% relative to the first MFI value and is at a level that a physician considers would be unsafe for cell transplantation (preferably >1000, more preferably >2000), this indicates that the patient is sensitized and the conditioning regimen should be used.
(B) First MFI value between 1000 and 10,000 for at least one HLA molecule present in the donor, plus the corresponding second MFI value is not reduced by at least 50% relative to the first MFI value, this indicates that the patient is sensitized and the conditioning regimen should be used, optionally including a higher initial dose of IgG-inactivating enzyme;
(C) First MFI value >10,000 for at least one HLA molecule present in the donor indicates that the patient is sensitized and the conditioning regimen should be used, optionally including a higher initial dose of IgG-inactivating enzyme.

13. A method for the prevention or treatment of immune rejection of a cell transplant to a sensitized subject, the method comprising administering a cell transplant to the subject in accordance with the conditioning regimen of any one of claims 1 to 12.

14. A method for the prevention or treatment of engraftment failure or poor graft function of a cell transplant to a sensitized subject, the method comprising administering a cell transplant to the subject in accordance with the conditioning regimen of any one of claims 1 to 12.

15. A method for the prevention or treatment of a disease or condition in a sensitized subject, which disease or condition is prevented or treated by cell transplant, the method comprising administering a cell transplant to the subject in accordance with the conditioning regime of any one of claims 1 to 12, optionally wherein the cell transplant is of HSPC and/or wherein the disease or condition is selected from:
- Hematological malignancies such as leukemias (for example Acute lymphoblastic leukemia (ALL), Acute myeloid leukemia (AML), Chronic lymphocytic leukemia (CLL), Chronic myelogenous leukemia (CML); lymphomas (for example Hodgkin's disease, Non-Hodgkin's lymphoma) and Myelomas (for example, Multiple myeloma (Kahler's disease));
- Hematologic diseases, such as phagocyte disorders (for example Myelodysplasia) or Hemoglobinopathies (for example Sickle cell disease, β thalassemia major (Cooley's anemia);
- Anemias (for example Paroxysmal nocturnal hemoglobinuria (PNH; severe aplasia), Aplastic anemia, Acquired pure red cell aplasia, Diamond-Blackfan anemia, Fanconi anemia); Hemoglobinopathies (for example Sickle cell disease, β thalassemia major (Cooley's anemia), Cytopenias (for example Amegakaryocytic thrombocytopenia); and Hemophagocytic syndromes (for example Hemophagocytic lymphohistiocytosis (HLH);
- Myeloproliferative disorders (for example Polycythemia vera, Essential thrombocytosis, Myelofibrosis);
- Solid tumor cancers, such as Neuroblastoma, Desmoplastic small round cell tumor, Ewing's sarcoma, Choriocarcinoma;
- Metabolic disorders such as amyloidosis (for example Amyloid light chain (AL) amyloidosis);
- Environmentally-induced diseases such as radiation poisoning;
- Viral diseases such as Human T-lymphotropic virus (HTLV) or Human Immunodeficiency Viruses (HIV);
- Autoimmune diseases, such as multiple sclerosis;
Lysosomal storage disorders such as Lipidoses - disorders of lipid storage - (for example Neuronal ceroid lipofuscinoses, Infantile neuronal ceroid lipofuscinosis (INCL, Santavuori disease,), Jansky-Bielschowsky disease (late infantile neuronal ceroid lipofuscinosis)); Sphingolipidoses (for example Niemann-Pick disease, Gaucher disease); Leukodystrophies (for example Adrenoleukodystrophy, Metachromatic leukodystrophy, Krabbe disease globoid cell leukodystrophy)); Mucopolysaccharidoses (for example Hurler syndrome (MPS I H, α-L-iduronidase deficiency), Scheie syndrome (MPS I S), Hurler-Scheie syndrome (MPS I H-S), Hunter syndrome (MPS II, iduronidase sulfate deficiency), Sanfilippo syndrome (MPS III), Morquio syndrome (MPS IV), Maroteaux-Lamy syndrome (MPS VI), Sly syndrome (MPS VII)); Glycoproteinoses (for example Mucolipidosis II (I-cell disease), Fucosidosis, Aspartylglucosaminuria, Alpha-mannosidosis; or Others (for example Wolman disease (acid lipase deficiency);
- Immunodeficiencies, such as T-cell deficiencies (for example Ataxia-telangiectasia, DiGeorge syndrome); Combined T- and B-cell deficiencies (for example Severe combined immunodeficiency (SCID), all types); Wiskott-Aldrich syndrome; Phagocyte disorders (for example Kostmann syndrome, Shwachman-Diamond syndrome); Immune dysregulation diseases (for example Griscelli syndrome, type II); Innate immune deficiencies (for example NF-Kappa-B Essential Modulator (NEMO) deficiency.
